# EUROPEAN PATENT SPECIFICATION

(11) **EP 3 363 372 B1**
(45) Date of publication and mention of the grant of the patent: **01.04.2026**
(21) Application number: 16854766.9
(22) Date of filing: 29.06.2016
(51) Int. Cl.: A61B 17/072, A61B 17/29, A61B 17/00

(54) **SURGICAL INSTRUMENT HAVING BENDABLE ACTUATOR**
CHIRURGISCHES INSTRUMENT MIT BIEGBAREM AKTUATOR
INSTRUMENT CHIRURGICAL AVEC ACTIONNEUR FLEXIBLE

(30) Priority: 16.10.2015 CN 201510672007
(43) Date of publication of application: 22.08.2018
(73) Proprietor: Yisi Suzhou Medical Technology Co., Ltd., Suzhou City, Jiangsu 215163 (CN); Ezisurg Medical Co., Ltd., Shanghai 201203 (CN)
(72) Inventor: NIE, Honglin, Shanghai (CN); YANG, Guang, Shanghai (CN); ZHANG, Xiliang, Shanghai (CN); JIANG, Yang, Shanghai (CN); SHI, Xiufeng, Shanghai (CN); LI, Anhua, Shanghai (CN)
(74) Representative: Manitz Finsterwald Patent- und Rechtsanwaltspartnerschaft mbB
(86) International application number: PCT/CN2016/087641
(87) International publication number: WO 2017/063382

(56) References cited:
- EP-A2- 0 717 959
- CN-A- 1 720 868
- CN-A- 1 993 083
- CN-A- 101 594 816
- CN-A- 102 469 997
- CN-A- 103 181 808
- CN-A- 105 434 002
- CN-U- 205 339 036
- US-A- 5 405 344
- US-A1- 2012 310 220
- US-A1- 2013 126 586
- US-A1- 2013 274 722
- US-A1- 2014 303 668

## Description

### BACKGROUND OF THE INVENTION

### Technical Field

The invention relates to a medical instrument, in particular to an endoscope instrument.

### Description of Related Art

A surgical stapler is a surgical instrument. According to the action principle of the surgical stapler, two corresponding actuators (generally comprising an anvil assembly and a cartridge assembly) are closed to clamp tissue, and metal suturing staples in the cartridge of the stapler are then pushed out to suture tissue. Certain staplers are further provided with a cutter used for incising sutured tissue.

With the advances in technology, traditional surgery methods are gradually being replaced by endoscope surgery. In endoscope surgery, a plurality of small incisions with a diameter of 5-12mm are punctured at different positions in the abdomen or the chest, a camera lens and various special surgical instruments are inserted into the abdomen cavity through these incisions, images taken by the camera inserted into the abdomen cavity of various visceral organs in the abdomen cavity are transmitted to a video screen to be observed by a surgeon, and the surgery is completed through extracorporeal operation by means of various surgical instruments. An endoscope stapler plays a key role in the surgery.

However, endoscope surgery also has limitations. As the space in the abdomen cavity or the thoracic cavity is limited, traditional linear endoscope staplers cannot effectively reach the surgical site to clamp, transect and staple tissue under certain extreme conditions, and so endoscope staplers provided with bendable actuators have been created.

The Endo GIA Universal stapler and magazine of American Tyco Healthcare Company (later renamed as Covidien) are typical products with the above function. The product of the patent application No. US2010/0237131A1 to Tyco Healthcare adopts a hinge-eccentric rigid connecting rod hinge structure to achieve deflection of an actuator. Limited by the structural design principle, the maximum bending angle of the actuator of the magazine is only 45°. In surgery requiring an actuator to bend by a large angle, certain body parts cannot be removed using an actuator only capable of bending by a small angle. For instance, in low rectal rectectomy, as the entry angle of an instrument is limited by the space of the pelvic cavity, although the actuator of the magazine bends, the actuator still cannot suture or dissect tissue perpendicular to the rectum in surgery, and consequentially, patients with tumors at low positions cannot be treated with the instrument.

Thus, in this field, a surgical instrument provided with an actuator capable of bending by a larger angle is needed to adapt to medical treatment under more complex clinic conditions.

US 2013/0274722 discloses an electromechanical surgical system having an instrument housing for connecting with a shaft assembly, a shaft assembly, and an end effector. The end effector is an articulating end effector and the system includes a cable tensioning system for tensioning the articulation cables. US 2012/0310220 discloses a surgical articulation assembly including a control assembly and an articulable portion. The surgical articulation assembly has a passage there-through for receiving a surgical object. The articulable portion includes at lest two segments capable of independent movement.

### BRIEF SUMMARY OF THE INVENTION

The present invention is defined by the features of the independent claim. Embodiments are given in the dependent claims.

In the surgical instrument of the invention, the flexible components are adopted to enable the actuator to bend relative to the far end of the extension tube, and thus the actuator is capable of bending by a larger angle to adapt to medical treatment under more complex clinic conditions. For instance, surgery can be performed on parts with limited spaces (such as the thoracic cavity and the pelvic cavity) more conveniently, and thus the problems which cannot be solved by other surgical instruments are solved. The surgical instrument provided with the actuator capable of bending by a large angle of the invention can achieve excision of a disease focus, tissue incision and suturing at various inaccessible positions and thus is beneficial to more patients. For instance, for a patient suffering from rectal cancer, the actuator of the surgical instrument is still capable of rotating to be perpendicular to the rectum when located at the lowermost position of the rectum, and thus more patients suffering from low rectal cancer can receive minimally invasive surgery.

Furthermore, the surgical instrument of the invention not only can be applied to surgery, but also can be applied to relevant treatment and diagnosis.

Furthermore, the invention can be applied not only to endoscope staplers, but also to closers, electric scalpels and other products.

### BRIEF DESCRIPTION OF THE SEVERAL VIEWS OF THE DRAWINGS

The drawings provided with the description show illustrative embodiments of the invention and are used for explaining the characteristics of the invention together with the summary of the invention mentioned above and the detailed description of the invention mentioned below.
FIG. 1 is a design diagram showing a perspective view of a surgical instrument in one embodiment of the invention.
FIG. 2 is an overall diagram of a magazine in a surgical instrument in one embodiment of the invention.
FIG. 3 is an exploded view of a magazine in a surgical instrument in one embodiment of the invention.
FIG. 4 is a diagram of an actuator, in a linear state, of a magazine in a surgical instrument in one embodiment of the invention.
FIG. 5 is a more detailed view of part of FIG. 4.
FIG. 6 is a diagram of an actuator, bending towards a first side, of a magazine in a surgical instrument in one embodiment of the invention
FIG. 7 is a more detailed view of part of FIG. 6.
FIG. 8 is a diagram of an actuator, bending towards a second side, of a magazine in a surgical instrument in one embodiment of the invention.
FIG. 9 is a usage diagram of a surgical instrument in one embodiment of the invention.

### DETAILED DESCRIPTION OF THE INVENTION

Various embodiments are described in detail with reference to the accompanying drawings. Where possible, identical reference signs are used for indicating identical or similar parts in the drawings. Specific embodiments and effects realized are only for illustration and do not limit the scope of the invention, which is defined by the claims.

The expression 'illustrative' in the description refers to 'being used as demonstrations or examples or for illustration'. Any 'illustrative' realization in the description is not definitely interpreted as being superior to or better than other realizations.

For a brief description, the instrument of the invention is explained with a stapler as an example. Those skilled in the field will understand that the surgical instrument provided with the bendable actuator of the invention is not limited to endoscope staplers. The technique of the invention can also be applied to other products such as closers and electric scalpels.

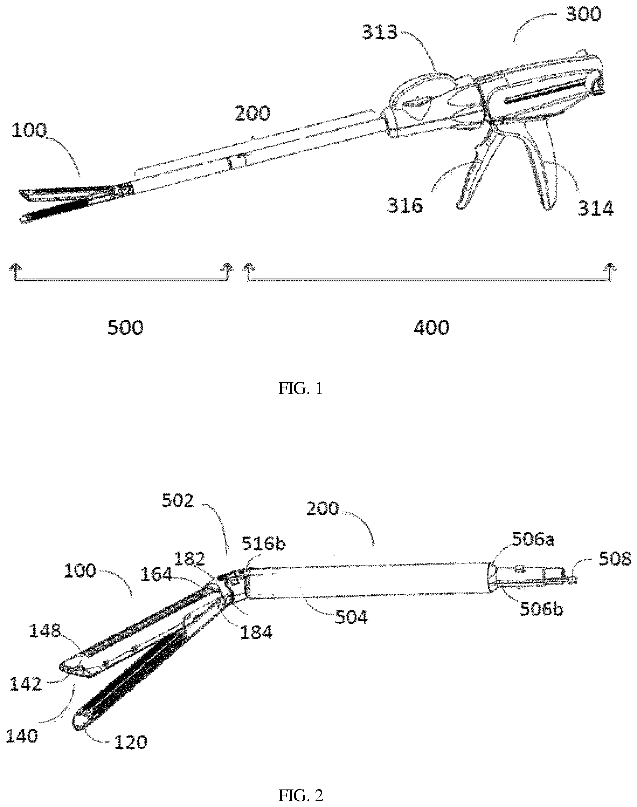

FIG. 1 is a design diagram of a surgical instrument in one embodiment of the invention. As is shown in FIG. 1, the surgical instrument comprises an actuator 100, an extension tube 200 and a controller 300. The extension tube 200 is movably connected with the far end of the controller 300 and connected with the near end of the actuator 100. The extension tube 200 forms a connecting passage and is used for transmitting the motion of the controller 300 to the actuator 100. The controller 300 is used for controlling operation of the instrument.

The surgical instrument of the invention can adopt an integrated design, namely, as a single structure, the extension tube 200 is connected with the far end of the controller 300 and the near end of the actuator 100. The length of the extension tube 200 can be selected according to different indications and specific conditions of patients.

As is shown in FIG. 1, the surgical instrument of the invention can also adopt a separable design. In the embodiment shown by FIG. 1, the surgical instrument adopting the separable design can comprise a stapler 400 and a magazine 500. In use, the stapler 400 and the magazine 500 can be assembled into a whole in a certain assembly manner. In the separable design, one stapler can be matched with various types of magazines in surgery.

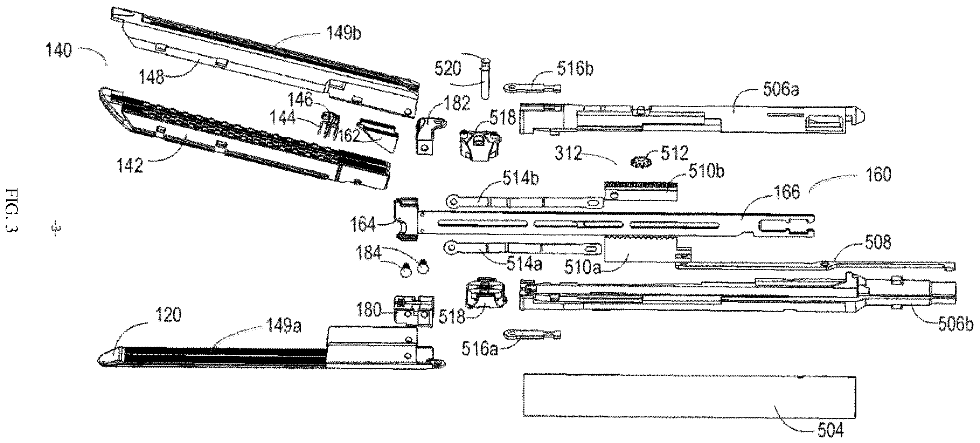

Referring to FIG. 1 and FIG. 3, the controller 300 can comprise a bend control mechanism 312. For instance, the bend control mechanism 312 can move in a first direction and in a second direction. As an example, the bend control mechanism 312 can be a gear reversing mechanism. The bend control mechanism 312 can comprise a gear 512, a first rack 510a and a second rack 510b. (It will be appreciated that the gear 512, the first rack 510a and the second rack 510b can also be disposed in the extension tube 200.) A handle component can be used as the controller 300. In this case, the controller 300 can further comprise a bend control grip 313, a fixed handle 314, a movable handle 316 (such as a percussion trigger) oppositely and movably connected with the fixed handle, and a transmission mechanism capable of transmitting the motion of the movable handle 316 to the actuator 100. For instance, in operation, the bend control grip 313 can be used to control the actuator 100 to rotate by a certain angle at the far end of the extension tube 200 and on the plane perpendicular to the hinge rotation axis. Every time the bend control grip 313 is moved to a specific position, the actuator 100 correspondingly rotates by a certain specific angle. The actuator 100 can rotate by different angles to adapt to different clinical applications. The percussion trigger 316 is pulled, and then the actuator 100 can continuously clamp, suture and cut human tissue. The function of using the bend control grip 313 to rotate the actuator 100 and the function of operating the percussion trigger 316 to drive the actuator to work are mutually independent. Generally, the operating process of the surgical instrument of the invention is as follows: the bend control grip 313 is operated first to control the actuator 100 to rotate to an angle suitable for a clinical application, the position of tissue in the jaw of the actuator 100 is then adjusted, and afterwards, the percussion trigger 316 is operated to achieve tissue clamping, suturing and incision.

A detailed description is given as follows with the endoscope stapler adopting the separated design as an example.

FIG. 2 and FIG. 3 respectively show the overall diagram and the exploded view of the magazine 500 in the surgical instrument in one embodiment of the invention.

In one embodiment of the invention, a bendable magazine 500 is provided. As an example, the magazine 500 can comprise a magazine body 501, an intermediate connecting part 502, an actuator 100 and a driving assembly 160.

As an example, the magazine body 501 can mainly comprise a magazine sleeve 504, a first magazine inner tube 506a, a second magazine inner tube 506b, a bend control rod 508, a first rack 510a, a second rack 510b, a gear 512, a first flexible pulling piece 514a, a second flexible pulling piece 514b, a first connecting piece 516a, a second connecting piece 516b, and the like. In the embodiment, the first magazine inner tube 506a, the second magazine inner tube 506b and the magazine sleeve 504 are arranged coaxially. The gear 512 is rotatably arranged on the first magazine inner tube 506a or the second magazine inner tube 506b. Although the FIGs show that separate magazine inner tubes such as the first magazine inner tube 506a and the second magazine inner tube 506b are adopted, it will be appreciated that in certain other embodiments, an integrated magazine inner tube is also available.

The intermediate connecting part 502 (shown in Fig 2) can comprise intermediate connectors 518 (two intermediate connectors are shown in FIG. 3) and a rivet 520.

The actuator 100 can comprise an anvil assembly 120 and a cartridge assembly 140 and can further comprise an anvil connector 180, a gland 182 and a rivet a 184. The anvil assembly 120 can comprise a staple forming face with a plurality of rows of staple forming slots. The cartridge assembly 140 can comprise a cartridge 142, one or more suturing staples 144, one or more staple pushing blocks 146 and a cartridge holder 148. The upper surface of the cartridge 142 is a tissue contact surface, and the cartridge 142 is mounted in the cartridge holder 148. The near end of the anvil assembly 120 is movably connected with the near end of the cartridge holder 148 of the cartridge assembly 140, and the anvil assembly 120 can be switched to be in an open state or a closed state. The driving assembly 160 is connected with the transmission mechanism and used for, for instance, converting the motion of the percussion trigger 316 into the closing, percussion or opening motion of the actuator 100. Generally, the anvil assembly 120 and the cartridge holder 148 can respectively comprise a longitudinal groove 149a and a longitudinal groove 149b, and the longitudinal grooves 149a, 149b allow the driving assembly 160 to pass through. When the driving assembly 160 moves towards the far end of the actuator 100 through the longitudinal groove 149a or the longitudinal groove 149b, the anvil assembly 120 and the cartridge assembly 140 can be driven to switch to a second state (for instance, the closed state) from a first state (for instance, the open state), a staple pushing slider 162 and the staple pushing block 146 are driven to eject the suturing staple 144 out, the suturing staple 144 is then formed in one staple forming slot in the staple forming surface of the anvil assembly 120, and vice versa. Thus, the actuator is driven by the driving assembly to switch to the first state or the second state.

Generally, the driving assembly 160 can comprise a slider 162, a cutter 164 and a flexible cutter bar 166. The cutter 164 is used for incising tissue between a plurality of rows of staple lines after the tissue is sutured by the suturing staple 144.

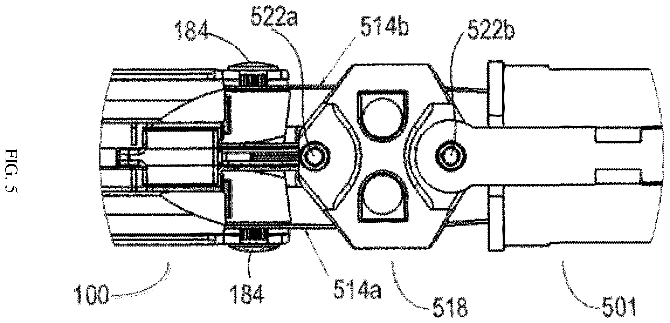

As shown in FIG 5, the magazine body 501, the intermediate connector 518 and the actuator 100 can be connected through one or more rotary hinges such as a hinge 522a and a hinge 522b. As an example, the two hinges 522a, 522b are spatially in parallel and perpendicular to the bendable plane of the actuator.

According to one example of the invention, the first magazine inner tube 506a and the second magazine inner tube 506b are coaxial with the magazine sleeve 504. The bend control rod 508 is arranged between the first magazine inner tube 506a and the second magazine inner tube 506b and fixedly connected with the first rack 510a. The first rack 510a and the second rack 510b are respectively arranged on the two sides of the first magazine inner tube 506a and are in engaged transmission through the gear 512 disposed on the rotation axis of the first magazine inner tube 506a. The first rack 510a is fixedly connected with the near end of the first flexible pulling piece 514a. The second rack 510b is fixedly connected with the near end of the second flexible pulling piece 514b. The first flexible pulling piece 514a and the second flexible pulling piece 514b penetrate through the intermediate connector 518 to be respectively connected to the left side and the right side of the central axis of the actuator 100.

According to one example of the invention, the flexible cutter bar 166 of the driving assembly 160 is arranged inside the magazine body 501 and penetrates through the intermediate connector 518. The cutter 164 and the slider 162 are arranged in the actuator 100.

When the magazine 500 is mounted on the stapler 400, the bend control rod 508 on the magazine 500 can be controlled to advance or retreat through the bend control mechanism 312 on the stapler 400.

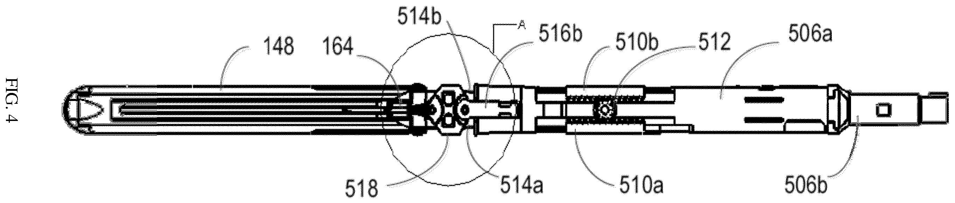

FIG. 4 and FIG. 5 respectively show a diagram and a local view A of the actuator, in a linear state, of the magazine in the surgical instrument in one embodiment of the invention.

As is shown in FIG. 4, according to one embodiment of the invention, the central axis of the actuator 100 is collinear with the central axis of the connecting mechanism (such as the magazine body or the extension tube). In this embodiment, the two flexible pulling pieces 514a, 514b are fixedly mounted on the rivet 184 of the actuator 100. Through the design, the joints of the flexible pulling pieces 514a, 514b and the actuator deviate from the central symmetry plane where the rotation axes of the two hinges 522a, 522b are located, and the flexible pulling pieces 514a, 514b do not pass through the center of the hinges. Thus, with the hinges as the rotation axes, the flexible pulling pieces provide certain rotation torque for the actuator to drive the actuator to rotate.

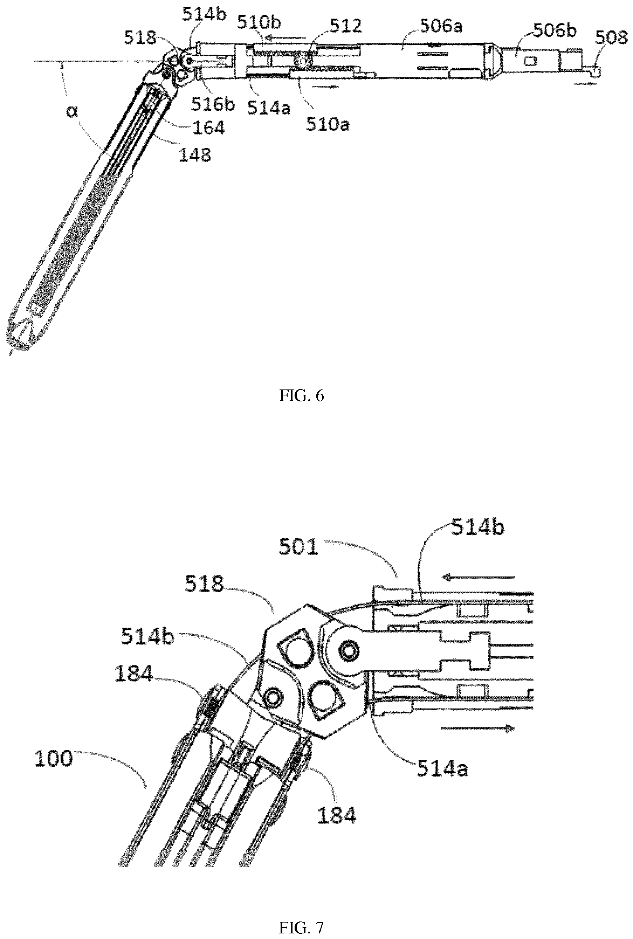

FIG. 6 and FIG. 7 respectively show a diagram and a local view of the actuator, bending towards a first side, of the magazine in the surgical instrument in one embodiment of the invention.

As is shown in the FIGs, when pulled, the bend control rod 508 pulls the first rack 510a to move towards the near end of the magazine 500 (as indicated by the arrow), and then the first rack 510a pulls the first flexible pulling piece 514a. At the moment, the first rack 510a drives the gear 512 to rotate, the second rack 510b is then driven to move towards the far end of the magazine 500 (as indicated by the arrow), and the second flexible pulling piece 514b is released. In this way, the actuator 100 of the magazine is bent toward the first side (for instance, in the anticlockwise direction shown in the FIGs). The rotation angle of the actuator 100 relative to the magazine body 501 is α. By controlling the feed amount of the bend control rod 508 through the bend control mechanism 312 on the stapler 400, the rotation angle α of the actuator 100 can be controlled. α can be any numerical value from 0° to 180° (such as 0°, 30°, 45°, 60°, 90°, 120°, 135° and 180°). According to the invention, α can be any numerical value from 0° to 135°.

As can be seen from the FIGs, the first flexible pulling piece is tensioned, the second flexible pulling piece is released, and thus the actuator is driven to bend. In this embodiment, the gear is engaged with the two racks, and the two racks move in opposite directions, so that when the actuator rotates, the displacement generated by the first tensioned flexible pulling piece and the displacement generated by the second released flexible pulling piece are equal and thus counteracted. In the design of the invention, the opposite displacements of the two flexible pulling pieces can also be counteracted through other methods.

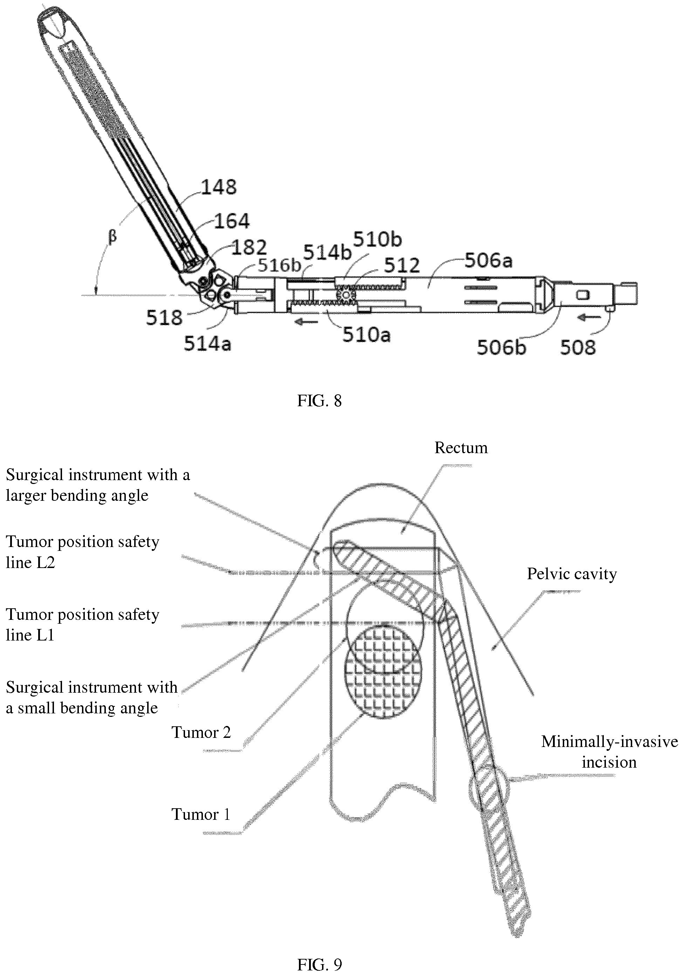

FIG. 8 shows a diagram of the actuator, bending towards a second side, of the magazine in the surgical instrument in one embodiment of the invention.

As is shown in the FIG, when pushed, the bend control rod 508 pushes the first rack 510a to move towards the far end of the magazine 500 (as indicated by the arrow), and the first flexible pulling piece 514a is released. The first rack 510a drives the gear 512 to rotate, the second rack 510b is then driven to move towards the near end of the magazine 500, the second rack 510b pulls the second flexible pulling piece 514b to make the actuator 100 of the magazine bend towards the second side (for instance, in the clockwise direction shown in the FIG), and the rotation angle is β. By controlling the feed amount of the bend control rod 508 through the bend control mechanism 312 on the stapler 400, the rotation angle β of the actuator can be controlled. β can be any numerical value from 0° to 180° (such as 0°, 30°, 45°, 60°, 90°, 120°, 135° and 180°). According to the invention, β can be any numerical value from 0° to 135°.

The principle for the actuator to bend towards the second side is similar to the principle for the actuator to bend towards the first side and is different in that the second flexible pulling piece is tensioned and the first flexible pulling piece is released.

FIG. 9 shows a usage diagram of the surgical instrument in one embodiment of the invention.

The surgical instrument of the invention can be applied to pulmonary surgery and digestive tract (comprising stomach and intestine) surgery. The pulmonary surgery is generally for excision of a disease focus. As for the digestive tract surgery, intestinal canals generally need to be reconnected after focus excision.

Generally, the actuator of the magazine of the bendable endoscope stapler can bend, the near end of the actuator is connected with the bend control mechanism in the controller (such as a stapler handle) through an intermediate mechanism (such as the bend control rod 508 in the FIGs), and the bend control mechanism can be adjusted to control the actuator to bend by multiple angles. In the initial state, the central axis of the actuator is collinear with the central axis of the connecting mechanism, the actuator of the stapler stretches into the pleural cavity or the abdominal cavity through a trocar, the actuator is controlled to bend by a certain angle through extracorporeal control over the bend control mechanism on the handle, and a series of operations such as clamping, transecting and suturing are performed on the surgical site. After the surgery is completed, the actuator is controlled to be in the linear state again through extracorporeal control over the bend control mechanism on the handle and then is pulled out of the body.

For instance, as for the digestive tract surgery, to completely remove a tumor and prevent tumor tissue residues in clinic, the transection line is generally about 5cm away from the boundary of the tumor. When a rectal cancer tumor is close to the lower portion of the anus of the patient, the actuator of the stapler needs to stretch to a lower position in the narrow pelvic cavity; however, as the entry position of the stapler is limited by the hip bone, an actuator of a stapler with a small bending angle cannot perpendicularly transect the rectum, and consequentially, the surgical risk of incomplete tumor excision is increased.

As can be seen from FIG 9, a surgical instrument (shown hatched) comprising an actuator with a small bending angle cannot perpendicularly transect the rectum, but a stapler (shown without hatching) comprising an actuator with a large bending angle can perpendicularly transect the rectum. Moreover, a surgical instrument comprising an actuator with a larger bending angle can transect a tumor (tumor 2) at a lower position. When the rotation angle of the actuator of the surgical instrument is further increased, a tumor at a lower position can be incised, and thus the surgical instrument can be well applied to low rectal cancer surgery or other conditions in which the in-vivo space is limited.

According to the surgical instrument of the invention, the rotation angle of the actuator can reach 90° or even 135° to adapt to extreme applications.

According to another embodiment of the invention, the flexible pulling pieces 514a, 514b can be replaced with wire ropes, such as single-strand wires, multi-strand intertwined wires or other flexible components. It should be understood that the flexible pulling pieces and the wire ropes are only for illustrative description and are not used for limiting the invention, and those skilled in the field can envisage other similar flexible components to realize bending of the actuator. These realization modes are also within the scope of the invention.

In addition, it also should be understood that all the embodiments described above can also be realized in surgical instruments such as integrally-designed endoscope staplers. Moreover, all the embodiments can also be realized in other products such closers and electric scalpels.

According to the surgical instrument of the invention, the flexible components are adopted to realize bending of the actuator relative to the far end of the extension tube so that the actuator can bend by a larger angle to adapt to medical treatment under more complex clinic conditions. For instance, surgery can be performed in parts with limited spaces (such as the thoracic cavity and the pelvic cavity) more conveniently, and thus the problems which cannot be solved by other surgical instruments are solved. The surgical instrument of the invention provided with the actuator capable of being bent by a large angle can achieve excision of a disease focus, tissue dissecting and suturing at various inaccessible positions, and thus is beneficial to more patients.

The above description and drawings are provided only for illustrative description. Any quotation about the singular forms of elements in the claims, such as the article 'one', 'certain' or 'said', should not be interpreted as the singular number of the elements. For each specific application, those skilled in the field can describe the structure in difference mode, and all these realization modes should fall within the scope of the invention.

Anyone skilled in the field can manufacture or use the invention based on the description before the embodiments disclosed. Various modifications of these embodiments can be easily obtained by those skilled in the field, and the general principle defined in the description can be applied to other embodiments without deviating from the scope of the invention. Thus, the invention is not limited to the embodiments given in the description, but should be granted the widest scope in accordance with the principle and novelties disclosed in the claims.

## Claims

1. A surgical instrument, comprising an actuator (100), an extension tube (200) and a controller (300), wherein
the near end of the extension tube (200) is connected with the controller (300), the far end of the extension tube (200) is connected with the actuator (100), and the actuator (100) is capable of rotating relative to the far end of the extension tube (200);
the controller (300) comprises a bend control mechanism (312) capable of moving in at least one direction;
the extension tube (200) comprises a first flexible component (514a), the first flexible component (514a) being capable of moving in at least one direction by movement of the bend control mechanism (312), and the first flexible component (514a) being capable of enabling the actuator (100) to rotate towards at least one side relative to the far end of the extension tube (200),
**characterized in that**
the far end of the extension tube (200) is connected with the actuator (100) through two and only two hinges (522a, 522b) being spatially in parallel and perpendicular to a bendable plane of the actuator (100), the first flexible component (514a) does not pass through the center of the two hinges (522a, 522b) so that the first flexible component (514a) can provide rotation torque for the actuator (100) with the two hinges (522a, 522b) as the rotation axes, and the actuator (100) is capable of being driven to rotate towards the at least one side relative to the far end of the extension tube (200); and
a rotation angle (α) of the actuator (100) towards the at least one side relative to the far end of the extension tube (200) is any numerical value from 0° to 135°, wherein the rotation angle (α) is the angle between the central axis of the extension tube (200) and the central axis of the actuator (100).

2. The surgical instrument according to Claim 1, **characterized in that**
the extension tube (200) further comprises a second flexible component (514b), and the far end of the first flexible component (514a) and the far end of the second flexible component (514b) are respectively connected to the actuator (100) on either side of the central axis of the actuator (100);
when moving in a first direction, the bend control mechanism (312) drives the first flexible component (514a) to move towards the near end of the bend control mechanism (312) and drives the second flexible component (514b) to move towards the far end of the bend control mechanism (312), so that the actuator (100) is driven to rotate towards a first side relative to the far end of the extension tube (200);
when moving in a second direction, the bend control mechanism (312) drives the first flexible component (514a) to move towards the far end of the bend control mechanism (312) and drives the second flexible component (514b) to move towards the near end of the bend control mechanism (312), so that the actuator (100) is driven to rotate toward a second side relative to the far end of the extension tube (200).

3. The surgical instrument according to Claim 2, **characterized in that**
the bend control mechanism (312) comprises a gear (512), a first rack (510a) and a second rack (510b);
the gear (512) is engaged with the first rack (510a) and the second rack (510b);
the first rack (510a) is connected with the near end of the first flexible component (514a), and the second rack (510b) is connected with the near end of the second flexible component (514b).

4. The surgical instrument according to Claim 2, **characterized in that**
the extension tube (200) further comprises a magazine sleeve (504), magazine inner tubes (506a, 506b), a bend control rod (508), a gear (512), a first rack (510a) and a second rack (510b), wherein
the magazine inner tubes (506a, 506b) and the magazine sleeve (504) are arranged coaxially;
the gear (512) is rotatably arranged on the magazine inner tubes (506a, 506b), the first rack (510a) and the second rack (510b) are arranged on the two sides of the gear (512), and the gear (512) is engaged with the first rack (510a) and the second rack (510b);
the bend control rod (508) is arranged in the magazine sleeve (504), the near end of the bend control rod (508) is connected with the bend control mechanism (312), the far end of the bend control rod (508) is fixedly connected with the first rack (510a), the first rack (510a) is further fixedly connected with the near end of the first flexible component (514a), and the second rack (510b) is fixedly connected with the near end of the second flexible component (514b);
the bend control mechanism (312) is capable of controlling movement of the bend control rod (508): when moving towards the near end of the bend control mechanism (312), the bend control rod (508) pulls the first rack (510a) and then pulls the first flexible component (514a), and the actuator (100) is pulled by the first flexible component (514a) to rotate towards a first side relative to the far end of the extension tube (200); and when moving towards the far end of the bend control mechanism (312), the bend control rod (508) pushes the first rack (510a) and then drives the second rack (510b) to move towards the near end through the gear (512) so as to pull the second flexible component (514b), and the actuator (100) is pulled by the second flexible component (514b) to rotate towards a second side relative to the far end of the extension tube (200).

5. The surgical instrument according to Claim 4, **characterized in that**
the magazine inner tubes (506a, 506b) include a first magazine inner tube (506a) and a second magazine inner tube (506b), the first magazine inner tube (506a) and the second magazine inner tube (506b) are coaxial with the magazine sleeve (504), and the gear (512) is rotatably arranged on the first magazine inner tube (506a) or the second magazine inner tube (506b).

6. The surgical instrument according to Claim 1, **characterized in that**
the actuator (100) comprises an anvil assembly (120) and a cartridge assembly (140), wherein the cartridge assembly (140) comprises a cartridge (142), one or more suturing staples (144), one or more staple pushing blocks (146), and a cartridge holder (148);
the surgical instrument further comprises a driving assembly (160), and the driving assembly (160) comprises a cutter (164) and a flexible cutter bar (166);
the near end of the anvil assembly (120) is movably connected with the near end of the cartridge holder (148), and the driving assembly (160) is capable of driving the actuator (100) to switch to a first state or a second state.

7. The surgical instrument according to any of Claims 2-5, **characterized in that**
at least one of the first flexible component (514a) or the second flexible component (514b) is a flexible pulling piece (514a, 514b) or a wire rope.

## Patentansprüche

1. Chirurgisches Instrument, umfassend einen Aktor (100), ein Verlängerungsrohr (200) und einen Controller (300), wobei
das nahe Ende des Verlängerungsrohrs (200) mit dem Controller (300) verbunden ist, das ferne Ende des Verlängerungsrohrs (200) mit dem Aktor (100) verbunden ist und der Aktor (100) in der Lage ist, sich relativ zu dem fernen Ende des Verlängerungsrohrs (200) zu drehen;
der Controller (300) einen Biegesteuermechanismus (312) umfasst, der in der Lage ist, sich in mindestens eine Richtung zu bewegen;
das Verlängerungsrohr (200) eine erste flexible Komponente (514a) umfasst, wobei die erste flexible Komponente (514a) in der Lage ist, sich durch Bewegung des Biegesteuermechanismus (312) in mindestens eine Richtung zu bewegen, und die erste flexible Komponente (514a) in der Lage ist, dem Aktor (100) zu ermöglichen, sich relativ zu dem fernen Ende des Verlängerungsrohrs (200) in Richtung mindestens einer Seite zu drehen,
**dadurch gekennzeichnet, dass**
das ferne Ende des Verlängerungsrohrs (200) mit dem Aktor (100) durch zwei und nur zwei Gelenke (522a, 522b) verbunden ist, die räumlich parallel und senkrecht zu einer biegbaren Ebene des Aktors (100) sind, die erste flexible Komponente (514a) nicht durch die Mitte der zwei Gelenke (522a, 522b) verläuft, so dass die erste flexible Komponente (514a) ein Drehmoment für den Aktor (100) mit den zwei Gelenken (522a, 522b) als die Drehachsen bereitstellen kann, und der Aktor (100) in der Lage ist, angetrieben zu werden, um sich relativ zu dem fernen Ende des Verlängerungsrohrs (200) in Richtung der mindestens einen Seite zu drehen; und
ein Drehwinkel (α) des Aktors (100) in Richtung der mindestens einen Seite relativ zu dem fernen Ende des Verlängerungsrohrs (200) ein beliebiger numerischer Wert von 0° bis 135° ist, wobei der Drehwinkel (α) der Winkel zwischen der Zentralachse des Verlängerungsrohrs (200) und der Zentralachse des Aktors (100) ist.

2. Chirurgisches Instrument nach Anspruch 1, **dadurch gekennzeichnet, dass**
das Verlängerungsrohr (200) ferner eine zweite flexible Komponente (514b) umfasst und das ferne Ende der ersten flexiblen Komponente (514a) und das ferne Ende der zweiten flexiblen Komponente (514b) jeweils mit dem Aktor (100) auf beiden Seiten der Zentralachse des Aktors (100) verbunden sind;
der Biegesteuermechanismus (312), wenn er sich in eine erste Richtung bewegt, die erste flexible Komponente (514a) antreibt, um sich in Richtung des nahen Endes des Biegesteuermechanismus (312) zu bewegen, und die zweite flexible Komponente (514b) antreibt, um sich in Richtung des fernen Endes des Biegesteuermechanismus (312) zu bewegen, so dass der Aktor (100) angetrieben wird, um sich relativ zu dem fernen Ende des Verlängerungsrohrs (200) in Richtung einer ersten Seite zu drehen;
der Biegesteuermechanismus (312), wenn er sich in eine zweite Richtung bewegt, die erste flexible Komponente (514a) antreibt, um sich in Richtung des fernen Endes des Biegesteuermechanismus (312) zu bewegen, und die zweite flexible Komponente (514b) antreibt, um sich in Richtung des nahen Endes des Biegesteuermechanismus (312) zu bewegen, so dass der Aktor (100) angetrieben wird, um sich relativ zu dem fernen Ende des Verlängerungsrohrs (200) in Richtung einer zweiten Seite zu drehen.

3. Chirurgisches Instrument nach Anspruch 2, **dadurch gekennzeichnet, dass** der Biegesteuermechanismus (312) ein Zahnrad (512), eine erste Zahnstange (510a) und eine zweite Zahnstange (510b) umfasst;
das Zahnrad (512) mit der ersten Zahnstange (510a) und der zweiten Zahnstange (510b) in Eingriff steht;
die erste Zahnstange (510a) mit dem nahen Ende der ersten flexiblen Komponente (514a) verbunden ist und die zweite Zahnstange (510b) mit dem nahen Ende der zweiten flexiblen Komponente (514b) verbunden ist.

4. Chirurgisches Instrument nach Anspruch 2, **dadurch gekennzeichnet, dass**
das Verlängerungsrohr (200) ferner eine Magazinhülse (504), Magazininnenrohre (506a, 506b), eine Biegesteuerstange (508), ein Zahnrad (512), eine erste Zahnstange (510a) und eine zweite Zahnstange (510b) umfasst, wobei
die Magazininnenrohre (506a, 506b) und die Magazinhülse (504) koaxial angeordnet sind;
das Zahnrad (512) drehbar auf den Magazininnenrohren (506a, 506b) angeordnet ist, die erste Zahnstange (510a) und die zweite Zahnstange (510b) auf den zwei Seiten des Zahnrads (512) angeordnet sind und das Zahnrad (512) mit der ersten Zahnstange (510a) und der zweiten Zahnstange (510b) in Eingriff steht;
die Biegesteuerstange (508) in der Magazinhülse (504) angeordnet ist, das nahe Ende der Biegesteuerstange (508) mit dem Biegesteuermechanismus (312) verbunden ist, das ferne Ende der Biegesteuerstange (508) fest mit der ersten Zahnstange (510a) verbunden ist, die erste Zahnstange (510a) ferner fest mit dem nahen Ende der ersten flexiblen Komponente (514a) verbunden ist und die zweite Zahnstange (510b) fest mit dem nahen Ende der zweiten flexiblen Komponente (514b) verbunden ist;
der Biegesteuermechanismus (312) in der Lage ist, die Bewegung der Biegesteuerstange (508) zu steuern: die Biegesteuerstange (508), wenn sie sich in Richtung des nahen Endes des Biegesteuermechanismus (312) bewegt, die erste Zahnstange (510a) zieht und dann die erste flexible Komponente (514a) zieht und der Aktor (100) durch die erste flexible Komponente (514a) gezogen wird, um sich relativ zu dem fernen Ende des Verlängerungsrohrs (200) in Richtung einer ersten Seite zu drehen; und die Biegesteuerstange (508), wenn sie sich in Richtung des fernen Endes des Biegesteuermechanismus (312) bewegt, die erste Zahnstange (510a) schiebt und dann die zweite Zahnstange (510b) antreibt, um sich durch das Zahnrad (512) in Richtung des nahen Endes zu bewegen, um die zweite flexible Komponente (514b) zu ziehen, und der Aktor (100) durch die zweite flexible Komponente (514b) gezogen wird, um sich relativ zu dem fernen Ende des Verlängerungsrohrs (200) in Richtung einer zweiten Seite zu drehen.

5. Chirurgisches Instrument nach Anspruch 4, **dadurch gekennzeichnet, dass**
die Magazininnenrohre (506a, 506b) ein erstes Magazininnenrohr (506a) und ein zweites Magazininnenrohr (506b) umfassen, das erste Magazininnenrohr (506a) und das zweite Magazininnenrohr (506b) koaxial zu der Magazinhülse (504) sind und das Zahnrad (512) drehbar auf dem ersten Magazininnenrohr (506a) oder dem zweiten Magazininnenrohr (506b) angeordnet ist.

6. Chirurgisches Instrument nach Anspruch 1, **dadurch gekennzeichnet, dass**
der Aktor (100) eine Ambossanordnung (120) und eine Kartuschenanordnung (140) umfasst, wobei die Kartuschenanordnung (140) eine Kartusche (142), eine oder mehrere Nähklammern (144), einen oder mehrere Klammerschiebeblöcke (146) und einen Kartuschenhalter (148) umfasst;
das chirurgische Instrument ferner eine Antriebsanordnung (160) umfasst und die Antriebsanordnung (160) eine Schneideinrichtung (164) und eine flexible Schneidstange (166) umfasst;
das nahe Ende der Ambossanordnung (120) beweglich mit dem nahen Ende des Kartuschenhalters (148) verbunden ist und die Antriebsanordnung (160) in der Lage ist, den Aktor (100) anzutreiben, um in einen ersten Zustand oder einen zweiten Zustand zu schalten.

7. Chirurgisches Instrument nach einem der Ansprüche 2-5, **dadurch gekennzeichnet, dass**
die erste flexible Komponente (514a) und/oder die zweite flexible Komponente (514b) ein flexibles Zugteil (514a, 514b) oder ein Drahtseil ist.

## Revendications

1. Instrument chirurgical, comprenant un actionneur (100), un tube d'extension (200) et un contrôleur (300), dans lequel
l'extrémité proche du tube d'extension (200) est connectée au contrôleur (300), l'extrémité éloignée du tube d'extension (200) est connectée à l'actionneur (100), et l'actionneur (100) est capable de tourner relativement à l'extrémité éloignée du tube d'extension (200) ;
le contrôleur (300) comprend un mécanisme de commande de courbure (312) capable de se déplacer dans au moins une direction ;
le tube d'extension (200) comprend un premier composant flexible (514a), le premier composant flexible (514a) étant capable de se déplacer dans au moins une direction via un déplacement du mécanisme de commande de courbure (312), et le premier composant flexible (514a) étant capable de permettre à l'actionneur (100) de tourner vers au moins un côté relativement à l'extrémité éloignée du tube d'extension (200),
**caractérisé en ce que**
l'extrémité éloignée du tube d'extension (200) est connectée à l'actionneur (100) par l'intermédiaire de deux et uniquement deux charnières (522a, 522b) qui sont parallèles et perpendiculaire dans l'espace à un plan de l'actionneur (100) pouvant être courbé, le premier composant flexible (514a) ne passant pas par le centre des deux charnières (522a, 522b) de telle sorte que le premier composant flexible (514a) peut fournir un couple de rotation pour l'actionneur (100) avec les deux charnières (522a, 522b) à titre d'axes de rotation, et l'actionneur (100) étant capable d'être entraîné pour tourner vers ledit au moins un côté relativement à l'extrémité éloignée du tube d'extension (200) ; et
un angle de rotation (α) de l'actionneur (100) vers ledit au moins un côté relativement à l'extrémité éloignée du tube d'extension (200) est une valeur numérique quelconque allant de 0° à 135°, l'angle de rotation (α) étant l'angle entre l'axe central du tube d'extension (200) et l'axe central de l'actionneur (100).

2. Instrument chirurgical selon la revendication 1, **caractérisé en ce que** le tube d'extension (200) comprend en outre un deuxième composant flexible (514b), et l'extrémité éloignée du premier composant flexible (514a) et l'extrémité éloignée du deuxième composant flexible (514b) sont respectivement connectées à l'actionneur (100) sur l'un ou l'autre côté de l'axe central de l'actionneur (100) ;
lors d'un déplacement dans une première direction, le mécanisme de commande de courbure (312) amène le premier composant flexible (514a) à se déplacer vers l'extrémité proche du mécanisme de commande de courbure (312) et amène le deuxième composant flexible (514b) à se déplacer vers l'extrémité éloignée du mécanisme de commande de courbure (312) de telle sorte que l'actionneur (100) est amené à tourner vers un premier côté relativement à l'extrémité éloignée du tube d'extension (200) ;
lors d'un déplacement dans une deuxième direction, le mécanisme de commande de courbure (312) amène le premier composant flexible (514a) à se déplacer vers l'extrémité éloignée du mécanisme de commande de courbure (312) et amène le deuxième composant flexible (514b) à se déplacer vers l'extrémité proche du mécanisme de commande de courbure (312), de telle sorte que l'actionneur (100) est amené à tourner vers un deuxième côté relativement à l'extrémité éloignée du tube d'extension (200).

3. Instrument chirurgical selon la revendication 2, **caractérisé en ce que**
le mécanisme de commande de courbure (312) comprend un pignon (512), une première crémaillère (510a) et une deuxième crémaillère (510b) ;
le pignon (512) est engagé avec la première crémaillère (510a) et avec la deuxième crémaillère (510b) ;
la première crémaillère (510a) est connectée à l'extrémité proche du premier composant flexible (514a), et la deuxième crémaillère (510b) est connectée à l'extrémité proche du deuxième composant flexible (514b).

4. Instrument chirurgical selon la revendication 2, **caractérisé en ce que**
le tube d'extension (200) comprend en outre un manchon de magasin (504), des tubes intérieurs de magasin (506a, 506b), une tige de commande de courbure (508), un pignon (512), une première crémaillère (510a) et une deuxième crémaillère (510b), dans lequel
les tubes intérieurs de magasin (506a, 506b) et le manchon de magasin (504) sont agencés de manière coaxiale ;
le pignon (512) est agencé en rotation sur les tubes intérieurs de magasin (506a, 506b), la première crémaillère (510a) et la deuxième crémaillère (510b) sont agencées sur les deux côtés du pignon (512), et le pignon (512) est engagé avec la première crémaillère (510a) et la deuxième crémaillère (510b) ;
la tige de commande de courbure (508) est agencé dans le manchon de magasin (504), l'extrémité proche de la tige de commande de courbure (508) est connectée au mécanisme de commande de courbure (312), l'extrémité éloignée de la tige de commande de courbure (508) est connectée de manière fixe à la première crémaillère (510a), la première crémaillère (510a) est en outre connectée de manière fixe à l'extrémité proche du premier composant flexible (514a), et la deuxième crémaillère (510b) est connectée de manière fixe à l'extrémité proche du deuxième composant flexible (514b) ;
le mécanisme de commande de courbure (312) est capable de commander un déplacement de la tige de commande de courbure (508) : lors d'un déplacement vers l'extrémité proche du mécanisme de commande de courbure (312), la tige de commande de courbure (508) tire la première crémaillère (510a) et puis tire le premier composant flexible (514a), et l'actionneur (100) est tiré par le premier composant flexible (514a) pour tourner vers un premier côté relativement à l'extrémité éloignée du tube d'extension (200) ; et lors d'un déplacement vers l'extrémité éloignée du mécanisme de commande de courbure (312), la tige de commande de courbure (508) pousse la première crémaillère (510a) et puis amène la deuxième crémaillère (510b) à se déplacer vers l'extrémité proche par l'intermédiaire du pignon (512) de manière à tirer le deuxième composant flexible (514b), et l'actionneur (100) est tiré par le deuxième composant flexible (514b) pour tourner vers un deuxième côté relativement à l'extrémité éloignée du tube d'extension (200).

5. Instrument chirurgical selon la revendication 4, **caractérisé en ce que**
les tubes intérieurs de magasin (506a, 506b) incluent un premier tube intérieur de magasin (506a) et un deuxième tube intérieur de magasin (506b), le premier tube intérieur de magasin (506a) et le deuxième tube intérieur de magasin (506b) sont coaxiaux par rapport au manchon de magasin (5041), et le pignon (512) est agencé en rotation sur le premier tube intérieur de magasin (506a) ou sur le deuxième tube intérieur de magasin (506b).

6. Instrument chirurgical selon la revendication 1, **caractérisé en ce que**
l'actionneur (100) comprend un assemblage formant enclume (120) et un assemblage formant cartouche (140), l'assemblage formant cartouche (140) comprenant une cartouche (142), une ou plusieurs agrafes de suture (144), un ou plusieurs blocs poussoirs d'agrafe, et un support de cartouche (148) ;
l'instrument chirurgical comprend en outre un assemblage d'entraînement (160), et l'assemblage d'entraînement (160) comprend un couteau (164) et une barre de coupe flexible (166) ;
l'extrémité proche de l'assemblage formant enclume (120) peut être connectée de manière mobile à l'extrémité proche du support de cartouche (148), et l'assemblage d'entraînement (160) est capable d'amener l'actionneur (100) à commuter dans un premier état ou dans un deuxième état.

7. Instrument chirurgical selon l'une des revendications 2 à 5,
**caractérisé en ce que** l'un au moins du premier composant flexible (514a) ou du deuxième composant flexible (514b) est une pièce de traction flexible (514a, 514b) ou un câble métallique.
